# EUROPÄISCHE PATENTSCHRIFT

(11) **EP 0 678 288 B1**
(45) Veröffentlichungstag und Bekanntmachung des Hinweises auf die Patenterteilung: **24.09.1997**
(21) Anmeldenummer: 95105751.2
(22) Anmeldetag: 18.04.1995
(51) Int. Cl.: A61F 9/06

(54) **Blendschutzvorrichtung**
Device to protect against glare
Dispositif de protection contre l'éblouissement

(30) Priorität: 22.04.1994 CH 1249/94
(43) Veröffentlichungstag der Anmeldung: 25.10.1995
(73) Patentinhaber: XELUX HOLDING AG, 6300 Zug (CH)
(72) Erfinder: Gunz, Stefan, CH-8820 Wädenswil (CH); Gunz-Castelberg, Donata, CH-8820 Wädenswil (CH); Castelberg, Manfred, CH-8820 Wädenswil (CH)
(74) Vertreter: Ritscher, Thomas, Dr.

(56) Entgegenhaltungen:
- EP-A- 0 536 081
- DE-A- 3 536 678
- DE-A- 3 842 824
- US-A- 5 172 256
- PATENT ABSTRACTS OF JAPAN vol. 15, no. 281 (P-1228) (4809) 17. Juli 1991 & JP-A-03 098 019 (AJINOMOTO CO INC.) 23. April 1991

## Beschreibung

Die vorliegende Erfindung betrifft eine Blendschutzvorrichtung gemäss Oberbegriff des vorliegenden Anspruchs 1 wie sie aus der DE-A-3536678 bekannt ist.

Blendschutzvorrichtungen sind dem Fachmann in verschiedenen Anwendungsgebieten und entsprechend angepassten Bauweisen bekannt. Diese Blendschutzvorrichtungen weisen alle im wesentlichen eine Lichtfilteranordnung mit mindestens einem passiven oder aktiven Polarisator-Analysator-System auf und sind mit weiteren Filterelementen, insbesondere IR- und UV-Sperrfiltern ausgerüstet. Bei aktiven Systemen mit Flüssigkristallzellen oder anderen elektrooptischen Bauelementen, wie sie insbesondere bei Schweisserschutzmasken verwendet werden, finden sich ausgeklügelte elektronische Steuerungen, Sensoranordnungen, Regeleinrichtungen und Spannungsversorgungsschaltungen, um den verschiedenen Bedürfnissen und Anforderungen an die spezifische Verwendung des Lichtschutzfilters gerecht werden zu können.

So ist bspw. aus der DE-35'36'678 eine Schweisserschutzmaske mit einem elektrooptischen Lichtfiltersystem bekannt. Dabei ist das Sichtfenster mit einem besonders ausgebildeten Rahmen in der Schutzmaske befestigt und führen elektrische Verbindungskabel jeweils zu dem an der Innenseite der Schutzmaske einzeln befestigten Elektronikteil, Sensorelement und Stromversorgungsteil. Die Nachteile einer derartigen Anordnung zeigen sich bei deren Verwendung und im Einsatz unter Schlechtwetterbedingungen oder anderen rauhen Arbeitsumständen. So erweisen sich derartige Anordnungen als äusserst anfällig und lassen sich nur schlecht gegen Staub und Nässe schützen. Das Auswechseln verschmutzter oder in ihrer Funktion gestörter Sichtfenster ist aufwendig und für die praktische Arbeit hinderlich, d.h. auch kostspielig.

Es ist deshalb das Bestreben der modernen Lichtschutztechnik anwendungsunabhängige und universell verwendbare Blendschutzvorrichtungen zu schaffen. Insbesondere ist es Aufgabe der vorliegenden Erfindung, eine universell verwendbare Blendschutzvorrichtung zu schaffen, welche die Nachteile der bekannten Vorrichtung nicht aufweist und vor allem vor den die einwandfreie Funktionsfähigkeit beeinträchtigenden Umwelteinflüssen gut geschützt ist, in einfacher Weise auswechselbar ist und in mit herkömmlichen Dunkelglas ausgerüsteten schutzbrillen, Schutzmasken oder Schutzhelme einsetzen lässt.

Erfindungsgemäss wird diese Aufgabe durch eine Blendschutzvorrichtung mit den Merkmalen des Anspruchs 1 gelöst. Diese Blendschutzvorrichtung, im folgenden auch Monoblock genannt enthält nicht nur das Lichtschutzfiltersystem, sondern umfasst gleichzeitig auch die erforderliche Elektronik und Stromversorgung. Damit dieser Monoblock in einfacher Weise anstelle der, für besondere Arbeiten besser geeigneten Dunkelgläser eingesetzt werden kann, entspricht die Dicke und äussere Form der gesamten Blendschutzvorrichtung derjenigen dieser Dunkelgläser. Diese Monoblock-Bauweise, d.h. die gezielte Verwendung besonderer Dicht- oder Klebmassen ermöglicht erstmals auch einen sicheren Schutz des optischen Systems vor Feuchtigkeit, Staub oder anderen physikalischen resp. chemischen Verschmutzungen, erhöht die Lebensdauer und verringert die Störanfälligkeit der Blendschutzvorrichtung im praktischen Einsatz.

In einer bevorzugten Ausführungsform der erfindungsgemässen Blendschutzvorrichtung werden verschiedenartige Dichtungsmassen aufgetragen, insbesondere eingespritzt, vergossen oder angeschäumt.

Weitere Ausführungsformen der erfindungsgemässen Blendschutzvorrichtung ergeben sich aus den Merkmalen der vorliegenden Unteransprüchen.

So können bspw. die Dichtungsmassen mit Zusätzen versetzt sein, welche die Transmissionseigenschaften der Dichtmassen in gewünschter Weise beeinflussen oder in Kombination mit Beschichtungen der Deckplatten besondere optische Wirkungen zur Folge haben. Ebenso kann die Solarzelle Teil des Aufbaus der Lichtschutzfilteranordnung sein und werden die elektrischen Verbindungen mit elektrisch leitenden Dichtungsmassen erzeugt.

In einer weiteren Ausführungsform sind Bedienungs- und Einstellelemente vorgesehen, die bspw. in Form einer Folientastatur auf der Vorder- oder Rückseite der Blendschutzvorrichtung aufgebracht sein können. Es versteht sich, dass auch seitlich einsetzbare Batterien oder Steckkontakte für wiederaufladbare Spannungsquellen vorgesehen sein können.

Im folgenden soll die Erfindung an verschiedenen Ausführungsbeispielen und mit Hilfe der Figuren näher erläutert werden.

Es zeigen:
- Figur 1:: Einen Querschnitt durch eine erfindungsgemässe Blendschutzvorrichtung, welche eine Dichtmasse als passives optisches Filterelement aufweist;
- Figur 2:: Einen Querschnitt durch eine erfindungsgemässe Blendschutzvorrichtung, welche ein Trägerteil einer Flüssigkristallzelle als Deckplatte aufweist;
- Figur 3:: Einen Querschnitt durch eine erfindungsgemässe Blendschutzvorrichtung, welche eine Solarzelle als passives optisches Filterelement aufweist;
- Figur 4:: Einen Querschnitt durch eine erfindungsgemässe Blendschutzvorrichtung, bei welcher der Rand mit einem Kunststoffrahmen verkittet ist;
- Figur 5:: Einen Querschnitt durch eine erfindungsgemässe Blendschutzvorrichtung mit einer als Leiterplatte ausgebildeten Elektronik.

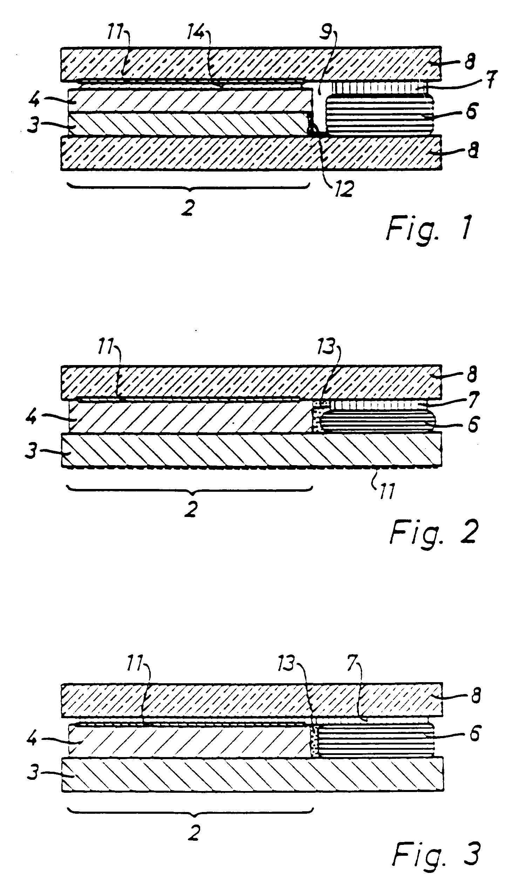

Die in Figur 1 dargestellte Blendschutzvorrichtung weist eine untere Deckplatte 8 aus Glas oder transparentem Polykarbonat auf. Darauf aufgeklebt ist eine erste Flüssigkristallzelle 3, welche mit Hilfe einer elektrisch leitenden Klebmasse 12 mit einem Elektronikbauteil 6 leitend verbunden ist. Eine unmittelbar auf die erste Flüssigkristallzelle 3 aufgesetzte zweite Flüssigkristallzelle 4 ist ebenfalls über eine elektrisch leitende Klebmasse 12 mit der Elektronik 6 verbunden. Der Raum zwischen den verschiedenen leitenden Klebmassen 12 kann mit einer isolierenden Dichtungsmasse 9 aufgefüllt sein. Die besondere Zusammensetzung der verwendeten Dichtunsmassen ist nicht Gegenstand der vorliegenden Erfindung und kann vom Fachmann in geeigneter Weise gewählt werden. Über den einzelnen Elektronikkomponenten und den Zwischenräumen 13 liegt eine Solarzelle 7, auf welche eine zweite obere Deckplatte 8 aufgesetzt ist. Diese obere Deckplatte 8 weist im Bereich der optischen Lichtschutzfilteranordnung 2 eine als IR-Sperrfilter wirkende Beschichtung 11 auf. Ein Zwischenraum zwischen dieser Beschichtung 11 und der zweiten Flüssigkristallzelle 4 ist mit einer mit optisch wirksamen Zusätzen versehenen Dichtungsmasse 14 aufgefüllt. Durch diese Aufbauweise erübrigen sich Halterungselemente, insbesondere Abstandshalter und lässt sich die Dicke der gesamten Blendschutzvorrichtung erheblich reduzieren.

Um jedoch einen erfindungsgemässen Monoblock mit geringer Dicke, in der Grössenordnung von 3 bis 5 mm realisieren zu können, weist die Ausführungsform gemäss Figur 2 anstelle der ersten Deckplatte 8 eine stabile LCD 3 auf, die aussenseitig mit einer zusätzlichen als Hitzereflektor oder Kratzschutz wirkenden, entspiegelnden und bruchsichernden Beschichtung 11 versehen ist. Ebenso ist die Elektronik 6 derart angeordnet, dass diese den zur Verfügung stehenden Raum in Längsrichtung vollumfänglich ausnutzt. Eine zweite LCD 4 ist wiederum über eine elektrisch leitende Klebmasse 12 mit dieser Elektronik 6 verbunden und liegt direkt unter einer optischen Sperrschicht 11 der oberen Deckplatte 8 aus Glas oder kratzfestem Kunststoff. Eine Solarzelle 7 zwischen der Elektronik 6 und dieser oberen Deckplatte 8 liefert den für die Steuerung und den Betrieb der LCD's 3, 4 notwendigen Strom.

Figur 3 zeigt eine Blendschutzvorrichtung, deren Aufbau sich von demjenigen aus Figur 2 nur dadurch unterscheidet, dass die Solarzelle 7 in einem definierten Mass transparent ist und über den optischen Bereich 2 des Lichtschutzfilters ragt und damit der Elektronik 6 eine leistungsfähigere Stromquelle zur Verfügung steht.

Die durch diese Monoblock-Bauweise erzielte Reduktion der Bauhöhe der Blendschutzvorrichtung wird im wesentlichen dadurch erreicht, dass die einzelnen elektro-optischen Elemente keine separaten Trägerelemente mehr benötigen.

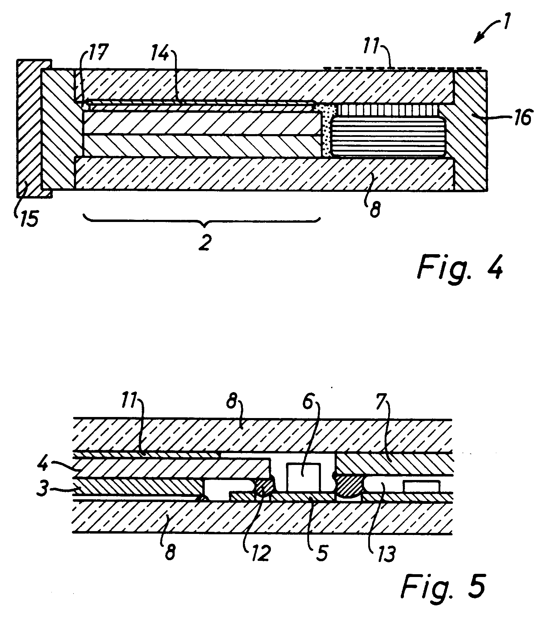

Aus Figur 4 ist die Verwendung einer Montagehilfe 16 ersichtlich, mit welcher der erfindungsgemässe Monoblock in einfacher Weise aufgebaut werden kann. Diese Montagehilfe 16 weist Positionierungsfinger 17 auf, welche die einzubauenden Elemente während der Montage in ihrer korrekten Stellung halten. Es versteht sich, dass mit dieser Monoblock-Bauweise Deckplatten 8 verwendet werden, auf welche die Elektronikschaltung in Form gedruckter Schaltungen 5 oder als Mikrochip direkt aufgebracht ist, oder mit Hilfe der Chip-on-glass-Technik aufgedampft und/oder aufdotiert ist, wie dies aus Figur 5 im Detail ersichtlich ist. Insbesondere kann die Elektronik 6 bei dieser Monoblock-Bauweise auch mit einer Folientastatur kombiniert werden, welche sowohl seitlich als auch in der Ebene der Deckplatten 8 angebracht werden kann. Die erfindungsgemäss verwendete Monoblocktechnik erlaubt auch Hohlräume bspw. für die Positionierung von auswechselbaren Zusatzbatterien zu schaffen.

Modifikationen und Weiterbildungen dieser Blendschutzvorrichtung liegen im Bereich des normalen fachmännischen Handelns und bedürfen kein besonderes erfinderisches Dazutun. Insbesondere können Farbgebungen oder Kombinationen bekannter mechanischer, optischer und elektro-optischer Elemente frei gewählt und zusammengestellt werden. Es versteht sich auch, dass diese erfindungsgemässe Blendschutzvorrichtung nicht nur als universelles Einsatzelement für Schweisserschutzmasken verwendet werden kann, sondern auch geeignet ist, um in Taucherbrillen, Pilotenhelmen oder Bergarbeitermasken eingesetzt werden zu können.

Die vorliegende Erfindung erlaubt auch einen spritzgegossenen Rahmen 15 zu verwenden, der die Blendschutzvorrichtung 1 vor mechanischen Beschädigungen schützt und in einfacher Weise so ausgeformt werden kann, dass sich diese formschlüssig in bestehende Halterungen einsetzen lässt. Es versteht sich auch, dass dieser Rahmen 15 und die Deckplatten 8 aus dem gleichen Material bestehen und aus einer allseitig spritzgegossenen Ummantelung gebildet sein können.

Ebenso liegt es im Bereich des fachmännischen Könnens, die eingegossene Elektronik mit Sensoren zu bestücken über welche eine kontaktlose Programmierung oder Einstellung vor oder während des Betriebs durch das hermetisch geschlossene Gehäuse vorgenommen werden kann.

## Patentansprüche

1. Blendschutzvorrichtung für Schutzbrillen, Schutzhelme oder Schutzmasken mit einem Lichtschutzfilter (2) aus mindestens einem aktiven (3, 4) und einem passiven (11) optischen Filterelement und mit einer Elektronik (6) zur Steuerung des mindestens einen aktiven optischen Filterelementes (3,4), sowie mit mindestens einer Stromversorgung für die Elektronik (6) und das mindestens eine aktive optische Filterelement (3, 4) sowie mit zwei transparenten Deckplatten (8), dadurch gekennzeichnet, dass die gesamte Blendschutzvorrichtung (1) eine herkömmlichen passiven Dunkelgläsern entsprechende Form mit konstanter Dicke, von insbesondere 3mm bis 5mm, aufweist und mindestens die optischen Elemente (3, 4, 11) des Lichtschutzfilters (2), die Elektronik (6) und Solarzellen (7) zwischen den Deckplatten angeordnet und mittels einer Dichtungsmasse (9, 14) vergossen und insbesondere untereinander verklebt sind um diese vor Staub, Feuchtigkeit oder Nässe, abrasiven oder agressiven, gasförmigen, flüssigen oder festen Substanzen zu schützen.

2. Blendschutzvorrichtung nach Anspruch 1, dadurch gekennzeichnet, dass die Dichtungsmasse (14) Zusätze enthält, welche eine optische Wirkung und insbesondere eine bestimmte Farb-, Transmissions- oder Filterwirkung, auch im UV und IR, erzeugen.

3. Blendschutzvorrichtung nach einem der Ansprüche 1 und 2, dadurch gekennzeichnet, dass mindestens eine der Deckplatten (8) mindestens auf ihrer Innenseite eine Beschichtung (11) aufweist.

4. Blendschutzvorrichtung nach Anspruch 3, dadurch gekennzeichnet, dass diese Beschichtung (11) eine als passives optisches Element, insbesondere als Polarisator oder optischer Filter wirkende Beschichtung ist.

5. Blendschutzvorrichtung nach Anspruch 3, dadurch gekennzeichnet, dass die Beschichtung (11) auf der Aussenseite der Deckplatte (8) eine als Hitze-Reflektor oder mechanischer Schutz, insbesondere als Kratzschutz, wirkende und entspiegelnde Beschichtung ist.

6. Blendschutzvorrichtung nach einem der Ansprüche 1 bis 4, dadurch gekennzeichnet, dass die Elektronik (6) direkt auf die Deckplatte (8) aufgebracht ist und insbesondere aufgedampft oder aufdotiert ist.

7. Blendschutzvorrichtung nach einem der Ansprüche 1 bis 6, dadurch gekennzeichnet, dass die Stromversorgung, Elektronik (6) und aktiven optischen Filterelemente (3, 4) mittels einer elektrisch leitenden Klebmasse (12) elektrisch miteinander verbunden sind.

8. Blendschutzvorrichtung nach einem der Ansprüche 1 bis 7, dadurch gekennzeichnet, dass die transparente Deckplatte (8) eine Glas- oder Kunststoffplatte ist.

9. Blendschutzvorrichtung nach Anspruch 8, dadurch gekennzeichnet, dass die Deckplatte (8) Teil eines aktiven optischen Filterelementes (3, 4) ist.

10. Blendschutzvorrichtung nach einem der Ansprüche 1 bis 9, dadurch gekennzeichnet, dass die Solarzelle (7) transparenter Bestandteil des Lichtschutzfilters (2) ist.

11. Blendschutzvorrichtung nach einem der Ansprüche 1 bis 10, dadurch gekennzeichnet, dass diese mindestens eine auswechselbare oder über die Solarzelle (7) oder über Kontakte aufladbare Batterie aufweist.

12. Blendschutzvorrichtung nach einem der Ansprüche 1 bis 11, dadurch gekennzeichnet, dass die Blendschutzvorrichtung (1) eine Montagehilfe (16) aufweist.

13. Blendschutzvorrichtung nach einem der Ansprüche 1 bis 12, dadurch gekennzeichnet, dass die Blendschutzvorrichtung (1) in einem spritzgegossenen Rahmen (15) eingefasst ist.

14. Blendschutzvorrichtung nach Anspruch 13, dadurch gekennzeichnet, dass die Deckplatten (8) und der Rahmen (15) eine gemeinsame und insbesondere einteilige Ummantelung bilden.

15. Blendschutzvorrichtung nach einem der vorgehenden Ansprüche, dadurch gekennzeichnet, dass die Elektronik Sensorelemente umfasst, über welche eine kontaktlose Programmierung oder Einstellung vor oder während des Betriebs durch das hermetisch geschlossene Gehäuse bewirkt werden kann.

## Claims

1. Anti-glare device for protective glasses, protective helmets or protective masks with a glare protection filter (2), comprising at least one active (3, 4) and one passive (11) optical filter element and with electronics (6) for controlling the at least one active optical filter element (3, 4), further comprising at least one current supply means for the electronics (6) and the at least one active optical filter element (3, 4), further comprising two transparent cover plates (8), characterized in that the entire anti-glare device (1) has the shape of known passive dark-glasses with constant thickness, in particular 3 mm to 5 mm, and at least the optical elements (3, 4, 11) of the light protection filter (2), the electronics (6) and solar cells (7) are arranged between the cover plates and are cast and in particular are adhesively attached to each other by means of a sealing mass (9, 14), in order to protect these against dust, humidity or wetness, abrasive or agressive gaseous, liquid or solid substances.

2. Anti-glare device according to claim 1, characterized in that the sealing mass (14) contains additives which cause an optical effect, in particular a specific colouring, transmitting or filtering effect, also in the UV- or IR-range.

3. Anti-glare device according to one of claims 1 and 2 characterized in that at least one of the cover plates (8) comprises one coating (11) at least on its interior surface.

4. Anti-glare device according to claim 3, characterized in that said coating (11) is a coating working as a passive optical element, in particular as a polarizer or optical filter.

5. Anti-glare device according to claim 3, characterized in that said coating (11) on the exterior side of the cover plate (8) is an anti-reflective coating working as a heat reflector or mechanical protection, in particular scratch protection.

6. Anti-glare device according to one of claims 1 to 4, characterized in that the electronics (6) are applied directly to the cover plate (8), and in particular are sputtered or doped on.

7. Anti-glare device according to one of claims 1 to 6, characterized in that the current supply, the electronics (6) and the active optical filter elements (3, 4) are electrically connected to each other by means of an electrically conductive adhesive material (12).

8. Anti-glare device according to one of claims 1 to 7, characterized in that the transparent cover plate (8) is a glass or plastic plate.

9. Anti-glare device according to claim 8, characterized in that the cover plate (8) is part of an active optical filter element (3, 4).

10. Anti-glare device according to one of claims 1 to 9, characterized in that the solar cell (7) is a transparent component of the light protection filter (2).

11. Anti-glare device according to one of claims 1 to 10, characterized in that said device comprises at least one replaceable battery and/or at least one battery which is rechargeable by means of the solar cell (7) or by means of contacts.

12. Anti-glare device according to one of claims 1 to 11, characterized in that the anti-glare device (1) comprises an assembly aid (16).

13. Anti-glare device according to one of claims 1 to 12, characterized in that the anti-glare device (1) is embedded in an injection-molded frame (15).

14. Anti-glare device according to claim 13, characterized in that the cover plates (8) and the frame (15) form a mutual and in particular a one-piece casing.

15. Anti-glare device according to one of the preceding claims, characterized in that the electronics comprise sensor elements, by means of which a contact-free programming or adjustment can be carried out prior to or during operation through the hermetically sealed casing.

## Revendications

1. Dispositif de protection contre l'éblouissement pour lunette de protection, casque de protection ou masque de protection présentant un filtre de protection optique (2) comprenant au moins un élément filtrant optique actif (3, 4) ou passif (11), et un circuit électronique (6) de commande d'au moins un élément filtrant optique actif (3, 4), tout comme au moins une alimentation électrique du circuit l'électronique (6) et d'au moins un élément filtrant optique actif (3, 4), tout comme deux plaques de recouvrement transparente (8), caractérisé en ce que l'ensemble du dispositif de protection contre l'éblouissement présente une forme d'épaisseur constante, en particulier de 3 à 5 mm, correspondant à la forme conventionnelle de verres fumés passifs, et en ce qu'au moins les éléments optiques (3, 4, 11) du filtre de protection optique (2), le circuit électronique (6) et une cellule solaire (7) sont agencés entre les plaques de recouvrement et moulés, en particulier collés entre-eux, au moyen d'une masse de remplissage (9, 14) afin de protéger ceux-ci de la poussière, de l'humidité et de substances abrasives ou agressives, gazeuses, fluides ou solides.

2. Dispositif de protection contre l'éblouissement selon la revendication 1, caractérisé en ce que la masse de remplissage (14) contient des additifs qui engendrent un effet optique et en particulier un effet particulier un effet ce couleur, de transmission ou de filtration, aussi dans la gamme des ultra-violets et des infra-rouges.

3. Dispositif de protection contre l'éblouissement selon l'une des revendications 1 ou 2, caractérisé en ce qu'au moins une des deux plaques de recouvrement (8) comprend un revêtement au moins sur sa face interne.

4. Dispositif de protection contre l'éblouissement selon la revendication 3, caractérisé en ce que ce revêtement (11) est un revêtement agissant en tant qu'élément optique passif, en particulier en tant que polariseur ou filtre optique.

5. Dispositif de protection contre l'éblouissement selon la revendication 3, caractérisé en ce que le revêtement (11) sur la face externe de la plaque de recouvrement (8) est un revêtement atténuant les réflexions et réfléchissant la chaleur ou de protection mécanique, en particulier de protection contre les éraflures.

6. Dispositif de protection contre l'éblouissement selon l'une des revendications 1 à 4, caractérisé en ce que le circuit électronique (6) est disposé directement, en particulier vaporisé ou dopé, sur la plaque de recouvrement (8).

7. Dispositif de protection contre l'éblouissement selon l'une des revendications 1 à 6, caractérisé en ce que l'alimentation électrique, le circuit électronique (6) et les éléments filtrants actifs optiques (3, 4) sont reliés électriquement entre-eux par l'intermédiaire d'une colle conduisant l'électricité.

8. Dispositif de protection contre l'éblouissement selon l'une des revendications 1 à 7, caractérisé en ce que la plaque de recouvrement transparente (8) est une plaque en verre ou en matière plastique.

9. Dispositif de protection contre l'éblouissement selon la revendication 8, caractérisé en ce que la plaque de recouvrement (8) fait partie d'un élément filtrant actif optique (3, 4).

10. Dispositif de protection contre l'éblouissement selon l'une des revendications 1 à 9, caractérisé en ce la cellule solaire (7) est un élément constitutif transparent du filtre de protection optique (2).

11. Dispositif de protection contre l'éblouissement selon l'une des revendications 1 à 10, caractérisé en ce que ce dispositif comprend au moins une batterie interchangeable ou rechargeable par la cellule solaire (7) ou par l'intermédiaire de contacts.

12. Dispositif de protection contre l'éblouissement selon l'une des revendications 1 à 11, caractérisé en ce que ce dispositif présente un support de montage (16).

13. Dispositif de protection contre l'éblouissement selon l'une des revendications 1 à 12, caractérisé en ce que ce dispositif est cerclé dans un cadre injecté (15).

14. Dispositif de protection contre l'éblouissement selon la revendication 13, caractérisé en ce que les plaques de recouvrement (8) et le cadre (15) constituent une gaine commune, en particulier d'une seule pièce.

15. Dispositif de protection contre l'éblouissement selon l'une des revendications précédentes, caractérisé en ce que le circuit électronique comprend des capteurs par l'intermédiaire desquels une programmation ou un réglage sans fil avant ou pendant l'utilisation peuvent être effectués au travers du bâti hermétiquement clos.
